# EUROPÄISCHE PATENTANMELDUNG

(11) **EP 1 013 336 A1**
(43) Veröffentlichungstag der Anmeldung: **28.06.2000**
(21) Anmeldenummer: 99121830.6
(22) Anmeldetag: 04.11.1999
(51) Int. Cl.: B01D 69/10, B01D 71/70, G01N 27/64

(54) **IMS-Membran mit gitterartiger Stützvorrichtung**

(30) Priorität: 24.12.1998 DE 19860265
(71) Anmelder: Bruker AG, 8117 Fällanden (CH)
(72) Erfinder: Grossnikolaus, Beat, Dipl. El. Ing. ETH, 8173 Riedt (CH)
(74) Vertreter: KOHLER SCHMID + PARTNER

(57) **Zusammenfassung**

Ein semipermeables Membranelement zur selektiven Zuführung von Messgas (6) in die Messzelle eines Ionen-Mobilitäts-Spektrometers (=IMS) ist dadurch gekennzeichnet, dass das semipermeable Membranelement ein tragendes, gitterartiges, gasdurchlässiges, vorzugsweise wabenförmiges Gerüst (2) aufweist, auf dem eine oder mehrere dünne Schichten (5) aus semipermeablen Materialien aufgebracht und mit dem Gerüst (2) mechanisch fest verbunden sind. Dieses Membranelement lässt sich in besonders einfacher, fertigungstechnisch unaufwendiger Weise mechanisch erheblich stabiler gestalten, ohne jedoch die Dicke der Membrane zu erhöhen und damit die erforderliche Reinigungszeit der Membrane sowie die Ansprechzeit des IMS-Spektrometers zu verlängern.

## Beschreibung

Die Erfindung betrifft ein semipermeables Membranelement zur selektiven Zuführung von Messgas in eine Messzelle eines Ionen-Mobilitätspektrometers (=IMS).

Ein solches Membranelement ist beispielsweise bekannt aus Ralph B. Spafford, "Evaluation of membranes for agent permeability" oder aus
Glenn E. Spangler, "Analysis of two membrane inlet systems on two potential trace vapor detectors", American Laboratory, 7 (July 1975),36-45.

Bei einem Ionen-Mobilitäts-Spektrometer (IMS) erfolgt die Zuführung des Messgases in die Messzelle im Allgemeinen durch eine semipermeable Membrane, die meistens aus Silikon (Poly-Di-Methyl-Siloxan) hergestellt und deshalb PDMS-Membrane genannt wird, und die eine Dicke von 10 bis 50 µm besitzt, vorzugsweise jedoch nur 20 bis 50 µm, da die Membrane, wenn sie zu dünn ist, sehr leicht reissen kann.

Die PDMS-Membran ist von Natur aus geringfügig gasdurchlässig und erzeugt damit die aus messtechnischen Gründen notwendige Trennung zwischen der Aussenluft, mit den zu messenden Gasanteilen, und dem Innern des Sensors. Daneben liefert sie auch einen natürlichen Schutz gegen gröbere Verunreinigungen in der Aussenluft. Sie besitzt aber auch die Eigenschaften eines Molekularfilters, indem sie einerseits ein unerwünschtes Gas, wie z.B. Wasserdampf, zurückhält, und anderseits nachzuweisende Gase, im besonderen die Phosphororganika, zu denen auch die chemischen Kampfgase gehören, durchlässt.

Die PDMS-Membrane weist aber auch einen gravierenden Nachteil auf, indem sie leider auch die Eigenschaft eines Schwammes besitzt, in welchem sich das zu messende Gas zum Teil verfängt und sich schlecht entfernen lässt. Das hat zur Folge, dass bei jedem neuen, empfindlichen Messvorgang, die PDMS-Membrane mit reiner Luft solange umspült werden muss, bis die Restanteile an Gasen der vorherigen Messung genügend gut entfernt sind. Die Zeit, die dabei verstreicht, nennt man die Reinigungszeit. Diese kann mehrere Minuten dauern und bewirkt eine massgebende Verzögerung der Messbereitschaft für die nächste Messung. Für eine PDMS-Membrane von ca. 30 µm dicke kann die Reinigungszeit 5 bis 8 Minuten betragen, wenn gewisse sehr schwer flüchtige Gase aus der Membrane entfernt werden sollen. Die Reinigungszeit ist abhängig von der Dicke der Membrane und ist umso kleiner, je dünner diese ist.

Die Bestrebungen sind deshalb gross, die Membrane so dünn wie möglich herzustellen, wobei heute Dicken von 20 bis 50 µm erreicht werden. Die Herstellung dieser dünnen Membranen ist jedoch sehr kritisch und führt im Allgemeinen zu grossen Ausschussraten, d.h. zu einer schlechten Produktionseffizienz. Bereits beim Trennen der hauchdünnen Membrane vom Stempel, mit dem sie erzeugt wurde, können Defekte entstehen. Die anschliessende Befestigung der Membrane am Befestigungsring ist ebenfalls sehr kritisch und führt zu weiteren Defekten. Alle diese Schwierigkeiten entstehen deshalb, weil diese Membrane sehr dünn, schwierig zu handhaben, und damit äusserst leicht zu zerstören ist.

Ein weiteres Problem bei diesen PDMS-Membranen ist die Einhaltung einer gleichmässigen Dicke. Eine gleichmässige Dicke ist aber unumgänglich, da die Membrane sonst an der dünnsten, und damit schwächsten Stelle, sofort reissen würde. Ausserdem muss die PDMS-Membrane im Betriebszustand des IMS-Spektrometers Druckunterschieden von bis zu 100 hPa standhalten können, ohne zu zerreissen. Auch dies ist eine harte Anforderung an die Festigkeit der Membrane und führt bei den herkömmlichen Membranen immer wieder zu Problemen.

Beim heutigen Stand der Technik steht man somit vor einem produktionstechnischen Problem, das die Herstellungskosten von PDMS-Membranen stark in die Höhe treibt. Es besteht deshalb ein starkes Bedürfnis nach zuverlässigeren Produktionsverfahren und mechanisch unempfindlicheren Membranen.

Eine weitere Grösse, welche die Leistungsfähigkeit eines IMS-Spektrometers massgebend bestimmt, ist dessen Ansprechzeit, d.h. die Zeitdifferenz zwischen dem Anbringen des Messgases auf der Aussenseite der Membrane und dem Erreichen eines IMS-Signals von 90% des Wertes, der nach genügend langem Abwarten erreicht wird. Diese Ansprechzeit sollte möglichst kurz sein, was wiederum eine möglichst dünne Membrane bedingt.

Aufgabe der vorliegenden Erfindung ist es daher, ein IMS-Membranelement der eingangs beschriebenen Art in möglichst einfacher, fertigungstechnisch unaufwendiger Weise mechanisch stabiler zu gestalten, ohne jedoch die Dicke der Membrane zu erhöhen und damit die erforderliche Reinigungszeit sowie die Ansprechzeit des IMS-Spektrometers zu verlängern.

Erfindungsgemäss wird diese Aufgabe auf ebenso überraschend einfache wie wirkungsvolle Art und Weise dadurch gelöst, dass das semipermeable Membranelement ein tragendes, gitterartiges, gasdurchlässiges Gerüst aufweist, auf dem eine oder mehrere dünnen Schichten aus semipermeablen Materialien aufgebracht und mit dem Gerüst mechanisch fest verbunden sind.

Die erfindungsgemässe Idee besteht im Wesentlichen darin, die Membrane mehrschichtig aufzubauen. Eine erste Schicht soll lediglich als Stütze für die nächste darauf liegende, semipermeable Schicht dienen, die für die eigentliche IMSMessung erforderlich ist. Die erste Schicht besteht aus einem gitterartigen Gerüst mit hoher Gasdurchlässigkeit. Die nächste Schicht besteht aus einer oder mehreren miteinander verbundenen Schichten aus semipermeablen Materialien, die mit der ersten Schicht, d.h. dem gitterartigen Gerüst, mechanisch fest verbunden sind.

Das gitterartige Gerüst übernimmt die Aufgabe, die dünnen und sehr empfindlichen semipermeablen Schichten zu stützen und damit zu verhindern, dass sie durch mechanische Einwirkungen zerstört werden. Das Gerüst darf aber auch kein Hindernis für den Durchgang des zu messenden Gases darstellen und muss deshalb gut gasdurchlässig sein.

In einer bevorzugten Ausführung besteht das gitterartige Gerüst aus einem wabenförmigen Gitter, insbesondere aus Nickel.

Damit kein merklicher Empfindlichkeitsverslust eintritt, sollte das Gitter das zu messende Gas möglichst gut durchlassen, d.h. das Verhältnis der Luftfläche zur Gesamtfläche des Gitters sollte möglichst nahe bei 100% liegen. Aus herstellungstechnischen Gründen liegen die Werte jedoch etwas tiefer, nämlich zwischen 50 und 100%, was noch immer hoch genug ist.

Bei einer bevorzugten Ausführungsform des erfindungsgemässen IMS-Membranelements beträgt der Maschenabstand ca. 400 µm und die Stegbreite ca. 80 µm, was zu einem Verhältnis von Luftfläche zu Gesamtfläche von ca. 64% führt.

Das wabenförmige Gitter lässt sich durch chemisches Ansetzen, d.h. Ausscheiden von Nickel, herstellen. Dieses Verfahren ist aus dem technischen Gebiet des Siebdruckes an sich bekannt, und zwar zur Herstellung der zum Druck benötigten Siebe.

Auf dem gitterartigen Gerüst ist eine semipermeable Membrane aufgebracht, die aus einer oder mehreren Schichten aufgebaut ist, z.B. aus dem bereits bekannten, reinen PDMS sowie aus funktionalen Polymerschichten mit selektiven Transportmechanismen.

In einer bevorzugten Ausführungsform besteht diese Membrane nur aus einer einzigen, dünnen, ca. 10 µm bis 50 µm, vorzugsweise 30 µm dicken Schicht aus aus Silikon, insbesondere aus PDMS.

Die Befestigung der Membran auf dem gitterartigen Gerüst geschieht bevorzugt mittels des an sich bekannten Transfer-Verfahrens, bei dem die noch nicht ganz auspolimerisierte und damit leicht klebrige PDMS-Membran durch Aufwalzen auf das gitterförmige Gerüst transferiert wird.

Eine IMS-Membrane, die nach der oben beschriebenen Methode hergestellt wird, besitzt eine viel höhere mechanische Festigkeit als alle herkömmlichen, ist reproduzierbar herzustellen und leichter zu handhaben, so dass eine hohe Produktionseffizienz verbunden mit einer hervorragenden Qualität erreicht wird.

Da die PDMS-Schicht durch das gitterförmige Gerüst mechanisch gestützt wird, lassen sich sogar Schichtdicken von bis gegen 10 µm realisieren, was bei den herkömmlichen Membranen aus Festigkeitsgründen nicht vertretbar wäre. Die mechanische Festigkeit der erfindungsgemässen Membrane ist zudem so gross, dass Druckdifferenzen von bis zu 100 hPa zwischen den beiden Seiten der Membrane zu keinerlei Zerreissproblemen führen, auch wenn die PDMS-Schichtdicke an der unteren Grenze liegt. Solche Druckdifferenzen können im Betriebszustand des IMS-Spektrometers auftreten.

Weitere Vorteile der Erfindung ergeben sich aus der Beschreibung und der Zeichnung. Ebenso können die vorstehend genannten und die noch weiter aufgeführten Merkmale erfindungsgemäss jeweils einzeln für sich oder zu mehreren in beliebigen Kombinationen Verwendung finden. Die gezeigten und beschriebenen Ausführungsformen sind nicht als abschliessende Aufzählung zu verstehen, sondern haben vielmehr beispielhaften Charakter für die Schilderung der Erfindung.

Die Erfindung ist in der Zeichnung dargestellt und wird anhand von Ausführungsbeispielen näher erläutert. Es zeigen:
- Fig.1: eine schematisierte Schrägsicht auf ein erfindungsgemässes IMS-Membranelement sowie eine Vergrösserung eines Flächenausschnittes daraus mit wabenförmiger Gitterstruktur; und
- Fig.2: einen schematisierten, stark vergrösserten Schnitt in der Ebene A-A durch das IMS-Membranelement nach Fig.1 mit angedeutetem Messgasstrom.

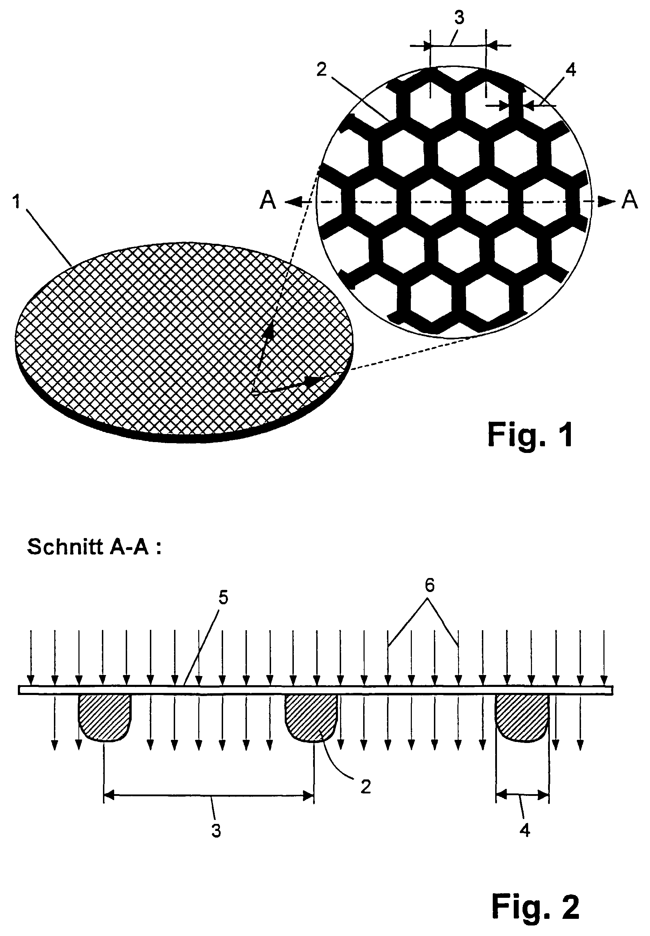

In Fig.1 ist in der linken unteren Bildhälfte ein semipermeables Membranelement 1 zur selektiven Zuführung von Messgas in eine in der Zeichnung nicht dargestellte Messzelle eines Ionen-Mobilitäts-Spektrometers (=IMS) gezeigt. Das IMS-Membranelement 1 besteht aus einem tragenden, gitterartigen Gerüst 2, welches aus einer wabenförmigen Struktur aus Nickel aufgebaut ist, die eine oder mehrere dünne Schichten aus semipermeablen Materialien trägt. In der rechten oberen Bildhälfte von Fig.1 in einem vergrößernden Ausschnitt näher dargestellt, weist die Wabenstruktur einen Maschenabstand 3 auf, der ca. 400 µm betragen kann, sowie eine Stegbreite 4, die im Ausführungsbeispiel ca. 80 µm beträgt. Das Verhältnis von Luftfläche zur Gesamtfläche des wabenförmigen Gitters 2 beträgt daher im gezeigten Beispiel ca. 64%.

Ein Schnitt durch das IMS-Membranelement 1 nach Fig.1 in Richtung A-A ist stark schematisiert in Fig.2 dargestellt:

Im unteren Bereich erkennt man die Stege des gitterartigen Gerüstes 2 mit Maschenabstand 3 und Stegbreite 4. Auf deren Oberseite ist eine semipermeable Schicht 5 mit einer Dicke 10 µm und 50 µm, vorzugsweise 30 µm mechanisch fest aufgebracht. Die semipermeable Schicht 5 kann aus einer einzigen Polymerschicht, beispielsweise aus Poly-Di-Methyl-Siloxan (= PDMS) oder aber auch aus mehreren reinen und/oder funktionalen Polymerschichten mit selektiven Transportmechanismen aufgebaut sein.

Die Gesamtpermeabilität des IMS-Membranelementes 1 wird, wie Fig.2 verdeutlicht, im wesentlichen durch die Permeabilität der permeablen Schicht(en) 5 bestimmt. Die durch Pfeile 6 angedeuteten Messgasströme werden nämlich von den Stegen des gitterartigen Gerüstes 2 nur zu einem sehr geringen Teil abgeblockt.

Die Herstellung des Wabengitters 2 erfolgt bevorzugt durch chemisches Ausscheiden von Nickel. Die semipermeablen Polymerschichten 5 können auf das Wabengitter 2 noch vor ihrem vollständigen Auspolymerisieren im Transfer-Verfahren aufgewalzt werden.

## Patentansprüche

1. Semipermeables Membranelement (1) zur selektiven Zuführung von Messgas (6) in eine Messzelle eines Ionen-Mobilitäts-Spektrometers (=IMS),
dadurch gekennzeichnet,
dass das semipermeable Membranelement (1) ein tragendes, gitterartiges, gasdurchlässiges Gerüst (2) aufweist, auf dem eine oder mehrere dünne Schichten (5) aus semipermeablen Materialien aufgebracht und mit dem Gerüst (2) mechanisch fest verbunden sind.

2. IMS-Membranelement nach Anspruch 1, dadurch gekennzeichnet, dass das gitterartige Gerüst (2) ein flächiges, wabenförmiges Gitter ist.

3. IMS-Membranelement nach Anspruch 2, dadurch gekennzeichnet, dass das wabenförmige Gitter aus Nickel aufgebaut ist.

4. IMS-Membranelement nach einem der vorhergehenden Ansprüche, dadurch gekennzeichnet, dass das gitterartige Gerüst (2) ein Verhältnis von Luftfläche zu Gesamtfläche grösser 1/2, vorzugsweise etwa 2/3 aufweist.

5. IMS-Membranelement nach einem der vorhergehenden Ansprüche, dadurch gekennzeichnet, dass das gitterartige Gerüst (2) einen Maschenabstand (3) von 250 bis 500 µm, vorzugsweise etwa 400µm, sowie eine Stegbreite (4) von 50 bis 100 µm, vorzugsweise etwa 80 µm aufweist.

6. IMS-Membranelement nach einem der vorhergehenden Ansprüche, dadurch gekennzeichnet, dass die semipermeablen Schichten (5) des IMS-Membranelements (1) eine Gesamtdicke von nicht mehr als etwa 50 µm, vorzugsweise zwischen 10 und 50 µm aufweisen.

7. IMS-Membranelement nach einem der vorhergehenden Ansprüche, dadurch gekennzeichnet, dass die semipermeablen Schichten (5) des IMS-Membranelements (1) aus reinen Polymerschichten und/oder aus funktionalen Polymerschichten mit selektiven Transportmechanismen bestehen.

8. IMS-Membranelement nach Anspruch 7, dadurch gekennzeichnet, dass das Ausgangsmaterial zur Herstellung der Polymerschichten Poly-Di-Methyl-Siloxan (=PDMS) ist.

9. IMS-Membranelement nach einem der vorhergehenden Ansprüche, dadurch gekennzeichnet, dass das IMS-Membranelement (1) nur eine einzige semipermeable Schicht (5) aufweist, die aus Silikon, insbesondere Poly-Di-Methyl-Siloxan (=PDMS) besteht.

10. IMS-Membranelement nach Anspruch 9, dadurch gekennzeichnet, dass die PDMS-Schicht eine Dicke von 10 bis 50 µm, vorzugsweise etwa 30 µm aufweist.

11. Verfahren zur Herstellung eines IMS-Membranelements (1) nach Anspruch 7 oder einem der Ansprüche 8 bis 10, dadurch gekennzeichnet, dass eine oder mehrere semipermeable Polymerschichten (5) noch vor ihrem vollständigen Auspolimerisieren im Transfer-Verfahren auf das gitterartige Gerüst (2) aufgebracht, vorzugsweise aufgewalzt werden.

12. Verfahren zur Herstellung eines IMS-Membranelements (1) nach einem der Ansprüche 1 bis 10, insbesondere auch nach Anspruch 11, dadurch gekennzeichnet, dass das gitterartige Gerüst (2) durch chemisches Ansetzen, insbesondere durch Ausscheiden von Nickel erzeugt wird.
